# FASCICULE DE BREVET EUROPEEN

(11) **EP 1 353 633 B1**
(45) Date de publication et mention de la délivrance du brevet: **28.05.2008**
(21) Numéro de dépôt: 02711923.9
(22) Date de dépôt: 04.01.2002
(51) Int. Cl.: A61K 8/35, A61K 8/81, A61Q 5/02, A61Q 5/06, A61Q 17/04, A61Q 19/00

(54) **COMPOSITIONS PHOTOPROTECTRICES A BASE DE POLYMERES AMPHIPHILES**
LICHTSCHUTZFORMULIERUNGEN MIT AMPHIPHILEN POLYMEREN
AMPHIPHILIC POLYMER-BASED PHOTOPROTECTIVE COMPOSITIONS

(30) Priorité: 11.01.2001 FR 0100334
(43) Date de publication de la demande: 22.10.2003
(73) Titulaire: L'ORÉAL, 75008 Paris (FR)
(72) Inventeur: BOUTELET, Karl, F-75011 Paris (FR); CANDAU, Didier, F-91570 Bièvres (FR)
(74) Mandataire: Miszputen, Laurent
(86) Numéro de dépôt international: PCT/FR2002/000030
(87) Numéro de publication internationale: WO 2002/055037

(56) Documents cités:
- EP-A- 0 406 042
- EP-A- 0 750 899
- EP-A- 0 815 843
- EP-A- 0 945 124
- EP-A- 1 055 406
- EP-A- 1 069 142
- WO-A-00/25731
- WO-A-00/31154
- WO-A-00/57893
- WO-A2-02/43689
- US-A- 4 460 732
- US-A- 4 861 499
- US-A- 5 089 578
- US-A- 5 114 706
- US-A- 5 464 452
- A KOBAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)
- R. LOCHEAD ET AL.: "Hydrophobically modified resins. A new route to easily prepared, storage-stable, cosmetic lotions that break upon contact with skin." SOAP/COSMETICS/CHEMICAL SPECIALTIES, mai 1987 (1987-05), pages 28,29,32,33,84-85, XP000929530

## Description

L'invention concerne des compositions cosmétiques à usage topique, en particulier pour la photoprotection de la peau et/ou des cheveux, caractérisées par le fait qu'elles comprennent, dans un support cosmétiquement acceptable (a) au moins un filtre UV organique et (b) au moins un polymère amphiphile comportant au moins un monomère à insaturation éthylènique à groupement sulfonique, sous forme libre ou partiellement ou totalement neutralisée et comprenant au moins une partie hydrophobe. L'invention concerne également leur application à la protection de la peau et des cheveux contre les effets du rayonnement ultraviolet.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques liposolubles et/ou des filtres organiques classiques hydrosolubles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras ; cependant, elles perdent également plus facilement leur efficacité en protection UV dès lors qu'elles viennent en contact avec l'eau ; en effet, les filtres hydrosolubles, ont tendance à disparaître à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

On peut disposer de compositions antisolaires présentant une résistance à l'eau améliorée en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

Ainsi, le besoin subsiste toujours quant à pouvoir disposer de compositions antisolaires apportant à la peau et/ou aux cheveux une protection solaire efficace, stable dans le temps et résistante à l'eau (rémanence à l'eau) et dont les performances cosmétiques seraient comparables à celles obtenues avec les émulsions huile/eau classiques.

On connaît dans l'article R.Y. Lochhead, W.J. Hemker et J.Y. Castaneda intitulé « Hydrophobically modified Carbopol Resins » extrait du journal Soap/Cosmetics/Chemical Specialities de mai 1987 des compositions solaires comprenant le polymère associatif acrylique modifié hydrophobe CARBOPOL 1342 et notamment un exemple avec des filtres organiques : benzophenone-3 et Octyl dimethyl PABA.

On connaît également dans la demande EP1069142 deux exemples 50 et 51 de gels capillaires comprenant 0,05% de benzophenone-4 et un copolymère réticulé obtenu par polymérisation d'acide 2-acrylamido-2-methylpropanesulfonique (AMPS) neutralisé à l'ammoniaque, d'ester d'acide méth(acrylique) et d'acide gras en C₁₆-C₁₈ de polyglycolether à 25 unités d'oxyde d'éthylène (25 OE) (GENAPOL T-250), de methylenbisacrylamide (réticulant), de t-butanol et d'azobisisobutyronitrile (initiateur).

On connaît également dans la demande EP1339383 deux exemples 8 et 9 de gels capillaires comprenant 0,05% de benzophenone-4 et respectivement
- un copolymère réticulé obtenu par polymérisation d'acide 2-acrylamido-2-methylpropanesulfonique (AMPS), de polyoxyethylen(10)behenylether (BB10), de Trimethylpropane Triacrylate et de poly(N-vinylpyrrolidone);
- un copolymère obtenu par réaction d'AMPS, d'Heptamethyltrisiloxane modifié polyoxyalkyléné (SILWET 7608), de Trimethylpropane Triacrylate et de poly(N-vinylformamide).

La Demanderesse a découvert de manière surprenante et inattendue que des compositions particulières contenant au moins un filtre UV organique et au moins un polymère amphiphile comportant au moins un monomère à insaturation éthylènique à groupement sulfonique, sous forme libre ou partiellement ou totalement neutralisée et comprenant au moins une partie hydrophobe permettaient non seulement d'obtenir des compositions antisolaires dont les performances cosmétiques étaient comparables à celles obtenues généralement avec une composition antisolaire classique sous forme d'émulsion huile/eau mais aussi présentaient un facteur de protection solaire (FPS) amélioré ainsi qu'une bonne rémanence à l'eau.

Ces découvertes sont à l'origine de la présente invention.

Ainsi, conformément à l'un des objets de la présente invention, il est maintenant proposé de nouvelles compositions cosmétique, en particulier pour la photoprotection de la peau et/ou des cheveux comprennant, dans un support cosmétiquement acceptable : (a) de 0,1 à 20% en poids par rapport au poids total de la composition d'au moins un filtre UV organique et (b) au moins un polymère amphiphile comportant au moins un monomère à insaturation éthylénique à groupement sulfonique, sous forme libre ou partiellement ou totalement neutralisée et en outre au moins une partie hydrophobe.

La présente invention a également pour objet l'utilisation de telles compositions pour la fabrication de compositions cosmétiques destinées à la protection de la peau et/ou des cheveux contre le rayonnement ultraviolet, en particulier le rayonnement solaire.

L'invention concerne aussi l'utilisation de cette composition pour la fabrication de compositions cosmétiques pour la protection de la peau et/ou des cheveux contre le rayonnement ultraviolet, en particulier le rayonnement solaire.

D'autres caractéristiques, aspects et avantages de la présente invention apparaîtront à la lecture de la description détaillée qui va suivre.

Les polymères conformes à l'invention sont des polymères amphiphiles comportant au moins un monomère à insaturation éthylènique à groupement sulfonique, sous forme libre ou partiellement ou totalement neutralisée et comprenant au moins une partie hydrophobe.

On entend par polymère amphiphile, tout polymère comportant à la fois une partie hydrophile et une partie hydrophobe et notamment une chaîne grasse.

La partie hydrophobe présente dans les polymères de l'invention comporte de préférence de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.
De préférence, on choisit des polymères amphiphiles réticulés.
Lorsqu'ils sont réticulés, les agents de réticulation peuvent être choisis parmi les composés à polyinsaturation oléfinique couramment utilisés pour la réticulation des polymères obtenus par polymérisation radicalaire.

On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétraallyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères à insaturation éthylènique à groupement sulfonique sont choisis notamment parmi l'acide vinylsulfonique, l'acide styrènesulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpéntane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.
Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- «Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704»;
- « Solution properties of miscelle networks formed by non-ionic moieties covalently bound to an polyelectrolyte : salt effects on rheological behavior - Langmuir, 2000, Vol.16, N°12, 5324-5332 »;
- « Stimuli responsive amphiphilic copolymers of sodium 2-(acrylamido)-2-methylpropanesulfonate and associative macromonomers - Polym. Preprint, Div. Polym. Chem. 1999,40(2),220-221».

Les monomères hydrophobes à insaturation éthylénique de ces copolymères particuliers sont choisis de préférence parmi les acrylates ou les acrylamides de formule (I) suivante : 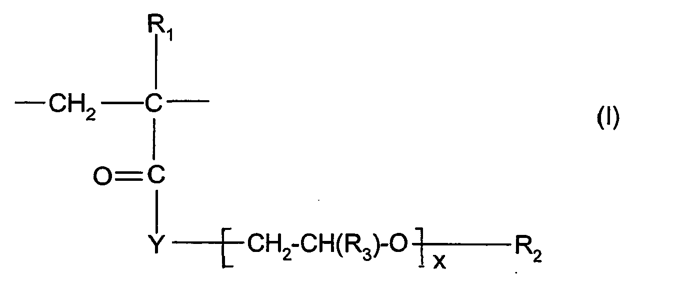 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de
carbone et plus préférentiellement de 6 à 22 atomes de carbone et encore plus préférentiellement de 6 à 18 atomes de carbone et plus particulièrement de 12 à 18 atomes de
carbone ; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)); les radicaux alkylperfluorés en C₆-C₁₈ (par exemple le groupement de formule -(CH₂)₂-(CF₂)₉-CF₃); le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (I) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0, 1 à 10% en mole de motifs AMPS et de 5 à 80% en mole de motifs n-(C₆-C₁₈)alkylacrylamide, tels que ceux décrits dans le brevet US- 5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (II) suivante : 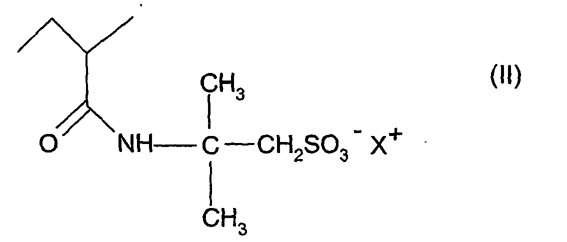 dans laquelle X⁺ est un proton, un cation de métal alcalin, un cation alcalino-terreux ou l'ion ammonium,
et de motifs de formule (III) suivante : 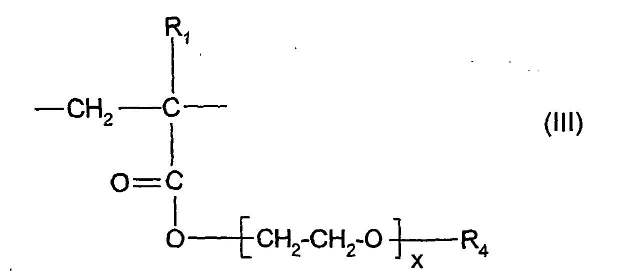 dans laquelle x désigne un nombre entier variant de 3 à 100, de préférence de 5 à 80 et
plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de Morishima mentionnés ci-dessus.

Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH ( 2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.
En utilisant la polymérisation par précipitation dans le tert-butanol, il est possible d'obtenir une distribution de la taille des particules du polymère particulièrement favorable pour ses utilisations.
La distribution de la taille des particules du polymère peut être déterminée par exemple par diffraction laser ou analyse d'image.
Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.
La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.
Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL® T-200 de la société HOECHST/CLARIANT),
- d'un alcool en C₁₆-C₁₈ oxyéthyléné par 25 moles d'oxyde d'éthylène (GENAPOL® T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.
De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.

De préférence pour les polymères peu hydrophobes, la proportion molaire en motifs de formule (I) ou (III) varie de 0,1 à 50%, plus particulièrement de 5 à 25% et encore plus particulièrement de 10 à 20%.
La distribution des monomères dans les polymères de l'invention peut être, par exemple, alternée, bloc (y compris multibloc) ou quelconque.
Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.
Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.
Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPa.s à 100 000 mPa.s et plus particulièrement de 60 000 mPa.s à 70 000 mPa.s.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

Les polymères amphiphiles utilisés selon l'invention peuvent, par exemple, être préparés comme suit :

### Préparation des esters (méth)acryliques éthoxylés

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite.
   Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote
ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.

De la façon décrite ci-dessus, on a préparé les polymères suivants :
(à partir des réactants suivants en des quantités exprimées en grammes)

| | Polymère P1 | Polymère P2 | Polymère P3 | Polymère P4 |
|---|---|---|---|---|
| Méthacrylate de Genapol T-250 | 10 | 20 | 30 | 97 |
| AMPS neutralisé par NH3 | 90 | 80 | 90 | 3 |
| Méthylène-bis-acrylamide (réticulant) | | | 1,5 | |
| Méthacrylate d'allyle (réticulant) | | 1,7 | | |
| TMPTA (réticulant) | 1,8 | | | 1,8 |
| Azobisisobutyronitrile (initiateur) | | | 1 | |
| Peroxyde dilauryle (initiateur) | 1 | 1 | | 1 |
| Tert-butanol | 300 | 300 | 300 | 300 |

Les polymères P1 et P2 sont utilisés dans les exemples.

Les filtres UV organiques conformes à l'invention sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649.

Comme exemples de filtres organiques complémentaires actifs dans l'UV-A et/ou l'UV-B, on peut citer désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques:

- Homosalate vendu sous le nom «EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS» par HAARMANN et REIMER,

### Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate vendu notamment sous le nom commercial « PARSOL MCX» par HOFFMANN LA ROCHE,
- Isopropyl Methoxy cinnamate,
- Isoamyl Methoxy cinnamate vendu sous le nom commercial «NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β'-diphénylacrylate :

- Octocrylene vendu notamment sous le nom commercial « UVINUL N539 » par BASF,
- Etocrylene, vendu notamment sous le nom commercial «UVINUL N35 » par BASF,

### Dérivés de la benzophénone :

- Benzophenone-1 vendu sous le nom commercial « UVINUL 400 » par BASF,
- Benzophenone-2 vendu sous le nom commercial « LTVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- 4-Metllylbenzylidene camphor vendu sous le nom « EUSOLEX 6300 » par MERCK,
- Benzylidene Camphor Sulfonic Acid fabriqué sous le nom «MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

### Dérivés de là triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,

### Dérivés du phenyl benzotriazole :

- Drometrizole Trisiloxane vendu sous le nom « SILATRIZOLE » par RHODIA CHIMIE,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés du benzalmalonate :

- Polyorganosiloxane à fonctions benzalmalonate vendu sous la dénomination commerciale « PARSOL SLX » par HOFFMANN LA ROCHE
et leurs mélanges.

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
et leurs mélanges.

Les filtres UV organiques sont présents dans les compositions selon l'invention dans des proportions allant de 0,1 à 20% en poids par rapport au poids total de la composition, et de préférence allant de 0,2 à 15% en poids par rapport au poids total de la composition.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les agents photoprotecteurs, les polymères autres que ceux de l'invention, les propulseurs, les agents alcalinisants ou acidifiants, les colorants, ou tout autre ingrédient habituellement utilisé dans le domaine cosmétique et/ou dermatologique, en particulier pour la fabrication de compositions autobronzantes sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir ce ou ces éventuels composés complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils
ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation.

MA signifie matière active.

Des exemples concrets, mais nullement limitatifs, illustrant l'invention, vont maintenant être donnés.

### EXEMPLES:

On réalise les compositions cosmétiques suivantes conforme à l'invention :

### EXEMPLE 1 : Shampooing

- Lauryl éther sulfate de Sodium 12 g MA vendu sous le nom d'EMPICOL ESB3/FL par la Société ALBRIGHT ET WILSON
- Benzophénone-1 0,5g
- Polymère P1 telle que défini dans la partie descriptive 1 g MA
- Acide citrique 3 g
- Eau pH ajusté à 4,8 (NaOH) qsp 100 g

Ce shampooing se présente sous l'aspect d'un liquide translucide épaissi et donne d'excellentes performances en terme de protection solaire.

### EXEMPLE 2 : Gel de coiffage

- Copolymère vinylpyrrolidone/diméthylamino éthylméthacrylate vendu sous le nom COPOLYMER 845 par la société ISP 1 gMA
- Benzophénone-4 1 g MA
- Polymère P2 préparé comme indiqué dans la partie descriptive 0,5 g MA
- Amino-2 méthyl-2 propanol-1 (AMP) pH ajusté à 7,5 qs
- Ethanol absolu 8,7 g
- Parfum, conservateur, colorant qs
- Eau déminéralisée qsp 100 g

On obtient un gel stable, épais, transparent, onctueux et homogène. Il donne d'excellentes performances en terme de protection solaire.

## Revendications

1. Composition cosmétique , en particulier pour la photoprotection de la peau et/ou des cheveux, **caractérisée par le fait qu'**elle comprend, dans un support cosmétiquement acceptable : (a) de 0,1 à 20% en poids par rapport au poids total de la composition d'au moins un filtre UV organique et (b) au moins un polymère amphiphile comportant au moins un monomère à insaturation éthylénique à groupement sulfonique, sous forme libre ou partiellement ou totalement neutralisée et en outre au moins une partie hydrophobe.

2. Composition selon la revendication 1, **caractérisée par le fait que** la partie hydrophobe du polymère amphiphile comporte de 6 à 50 atomes de carbone.

3. Composition selon la revendication 2, **caractérisée par le fait que** la partie hydrophobe du polymère amphiphile comporte de 6 à 22 atomes de carbone.

4. Composition selon la revendication 3, **caractérisée par le fait que** la partie hydrophobe du polymère amphiphile comporte de 6 à 18 atomes de carbone.

5. Composition selon la revendication 4, **caractérisée par le fait que** la partie hydrophobe du polymère amphiphile comporte de 12 à 18 atomes de carbone.

6. Composition selon l'une quelconque des revendications 1 à 5, **caractérisée par le fait que** les polymères amphiphiles sont neutralisés partiellement ou totalement par une base minérale ou organique.

7. Composition selon l'une quelconque des revendications 1 à 6, **caractérisée par le fait que** les polymères amphiphiles ont un poids moléculaire moyen en nombre allant de 1000 à 20 000 000 g/mole.

8. Composition selon la revendication 7, **caractérisée par le fait que** le poids moléculaire moyen en nombre varie de 20 000 à 5 000 000 g/mole.

9. Composition selon la revendication 8, **caractérisée par le fait que** le poids moléculaire moyen en nombre varie de 100 000 à 1 500 000 g/mole.

10. Composition selon l'une quelconque des revendications précédentes, **caractérisée par le fait que** les polymères amphiphiles sont préparés par polymérisation radicalaire par précipitation dans le tert-butanol.

11. Composition selon l'une quelconque des revendications précédentes, **caractérisée par le fait que** les polymères amphiphiles sont réticulés ou non-réticulés.

12. Composition selon la revendication 11, **caractérisée par le fait que** les polymères amphiphiles sont réticulés.

13. Composition selon la revendication 12, **caractérisée par le fait que** le ou les agents de réticulation sont choisis parmi les composés à polyinsaturation oléfinique.

14. Composition selon la revendication 13, **caractérisée par le fait que** le ou les agents de réticulation sont choisis parmi le méthylène-bis-acrylamide, le méthacrylate d'allyle ou le triméthylol propane triacrylate (TMPTA).

15. Composition selon l'une quelconque des revendications 12 à 14, **caractérisée par le fait que** le taux de réticulation varie de préférence de 0,01 à 10% en moles et plus particulièrement de 0,2 à 2% en moles par rapport au polymère.

16. Composition selon l'une quelconque des revendications précédentes, **caractérisée par le fait que** le monomère à insaturation éthylénique à groupement sulfonique est choisi parmi l'acide vinylsulfonique, l'acide styrène sulfonique, les acides (méth)acrylamido(C₁-C₂₂)alkylsulfoniques, les acides N-(C₁-C₂₂)alkyl(méth)acrylamido(C₁-C₂₂)alkylsulfoniques ainsi que leurs formes partiellement ou totalement neutralisées.

17. Composition selon la revendication 16, **caractérisée par le fait que** le monomère à insaturation éthylénique à groupement sulfonique est choisi parmi l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido 2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées.

18. Composition selon l'une quelconque des revendications 16 ou 17, **caractérisée par le fait que** le monomère à insaturation éthylénique à groupement sulfonique est l'acide 2-acrylamido-2-méthylpropane-sulfonique (AMPS), ainsi que ses formes partiellement ou totalement neutralisées.

19. Composition selon la revendication 18, **caractérisée par le fait que** les polymères amphiphiles sont choisis parmi les polymères statistiques d'AMPS modifiés par réaction avec une n-mono(C₆-C₂₂)alkylamine ou une di-n-(C₆-C₂₂)alkylamine.

20. Composition selon l'une quelconque des revendications 18 ou 19, **caractérisée par le fait que** les polymères amphiphiles d'AMPS contiennent en plus au moins un monomère à insaturation éthylénique ne comportant pas de chaîne grasse.

21. Composition selon la revendication 20, **caractérisée par le fait que** le monomère à insaturation éthylénique ne comportant pas de chaîne grasse est choisi parmi les acides (méth)acryliques et leurs dérivés alkyl substitués en β, et leurs esters obtenus avec des monoalcools ou des mono- ou polyalkylèneglycols, ou bien parmi les (méth)acrylamides, la vinylpyrrolidone , l'anhydride maléique, l'acide itaconique ou l'acide maléique, ou les mélanges de ces composés.

22. Composition selon l'une quelconque des revendications 1 à 18, **caractérisée par le fait que** les polymères amphiphiles d'AMPS sont choisis parmi les copolymères amphiphiles d'AMPS et d'au moins un monomère hydrophobe à insaturation éthylénique comportant au moins une partie hydrophobe ayant de 6 à 50 atomes de carbone.

23. Composition selon la revendication 22, **caractérisée par le fait que** la partie hydrophobe comporte de 6 à 22 atomes de carbone.

24. Composition selon la revendication 23, **caractérisée par le fait que** la partie hydrophobe comporte de 6 à 18 atomes de carbone.

25. Composition selon la revendication 24, **caractérisée par le fait que** la partie hydrophobe comporte de 12 à 18 atomes de carbone.

26. Composition selon l'une quelconque des revendications 22 à 25, **caractérisée par le fait que** le monomère hydrophobe à insaturation éthylénique est choisi parmi les acrylates ou les acrylamides de formule (I) suivante : 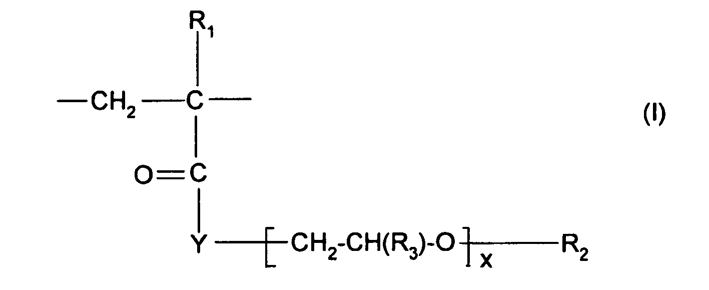 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

27. Composition selon la revendication 26, **caractérisée par le fait que** le radical hydrophobe R₂ est choisi parmi les radicaux alkyles en C₆-C₁₈, linéaires, ramifiés ou cycliques ; les radicaux alkylperfluorés en C₆-C₁₈; le radical cholestéryle ou un ester de cholestérol ; les groupes polycycliques aromatiques.

28. Composition selon l'une quelconque des revendications 26 ou 27, **caractérisée par le fait que** le monomère de formule (I) comporte en plus au moins un motif oxyde d'alkylène (x≥1).

29. Composition selon l'une quelconque des revendications 26 à 28, **caractérisée par le fait que** le monomère de formule (I) comporte en plus au moins une chaîne polyoxyalkylénée.

30. Composition selon la revendication 29, **caractérisée par le fait que** la chaîne polyoxyalkylénée est constituée de motifs oxyde d'éthylène et/ou de motifs oxyde de propylène.

31. Composition selon la revendication 30, **caractérisée par le fait que** la chaîne polyoxyalkylénée est constituée uniquement de motifs oxyde d'éthylène.

32. Composition selon l'une quelconque des revendications 26 à 31, **caractérisée par le fait que** le nombre de motifs oxyalkylénés varie de 3 à 100.

33. Composition selon la revendication 32, **caractérisée par le fait que** le nombre de motifs oxyalkylénés varie de 3 à 50.

34. Composition selon la revendication 33, **caractérisée par le fait que** le nombre de motifs oxyalkylénés varie de 7 à 25.

35. Composition selon l'une quelconque des revendications 22 à 27, **caractérisée par le fait que** le polymère amphiphile d'AMPS est choisi parmi :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₆₋₁₈)alkylacrylamide, par rapport au polymère.

36. Composition selon l'une quelconque des revendications 22 à 27, **caractérisée par le fait que** le polymère amphiphile d'AMPS est choisi parmi :
- les copolymères non réticulés d'AMPS partiellement ou totalement neutralisé et de méthacrylate de n-dodécyle.
- les copolymères réticulés ou non réticulés d'AMPS partiellement ou totalement neutralisé et de n-dodécylméthacrylamide.

37. Composition selon les revendications 22 à 34, **caractérisée par le fait que** le polymère amphiphile d'AMPS est choisi parmi les copolymères constitués de motifs acide 2-acrylamido-2-méthylpropane-sulfonique (AMPS) de formule (II) suivante : 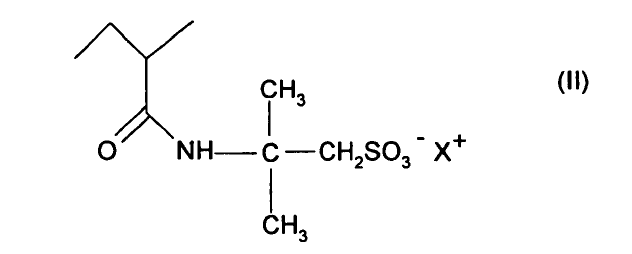 dans laquelle X⁺ est un proton, un cation de métal alcalin, un cation alcalino-terreux ou l'ion ammonium,
et de motifs de formule (III) suivante : 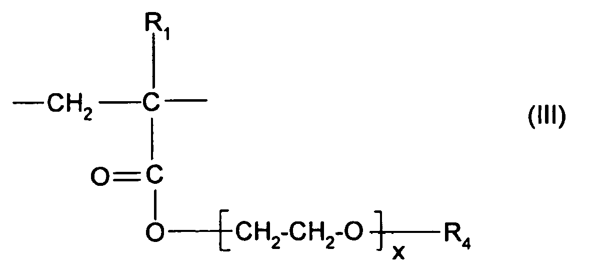 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

38. Composition selon la revendication 37, **caractérisée par le fait que** x = 25 , R₁ est méthyle et R₄ est n-dodécyle.

39. Composition selon la revendication 26 ou 37, **caractérisée par le fait que** la proportion molaire en % des motifs de formule (I) ou des motifs de formule (III) dans les polymères varie de 50,1 à 99,9%.

40. Composition selon la revendication 26 ou 37, **caractérisée par le fait que** la proportion molaire en % des motifs de formule (I) ou des motifs de formule (III) dans les polymères varie de 0,1 à 50%.

41. Composition selon l'une quelconque des revendications précédentes, **caractérisée par le fait que** les polymères amphiphiles sont présents dans des concentrations allant de 0,01 à 30% en poids, plus préférentiellement de 0,1 à 10% en poids, encore plus préférentiellement de 0,1 à 5% en poids, et plus particulièrement encore de 0,5 à 2% en poids par rapport au poids total de la composition.

42. Composition selon l'une quelconque des revendications 1 à 41, **caractérisée par le fait que** le ou les filtres UV organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate, les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène.

43. Composition selon la revendications 42, **caractérisée par le fait que** le ou les filtres UV organiques sont choisis parmi les composés suivants :
- PABA,
- Ethyl PABA,
- Ethyl Dihydroxypropyl PABA
- Ethylhexyl Diméthyl PABA
- Glycéryl PABA,
- PEG-25 PABA,
- Homosalate,
- Ethylhexyl Salicylate
- Dipropyleneglycol Salicylate,
- TEA Salicylate,
- Butyl Methoxydibenzoylméthane,
- Isopropyl Dibenzoylméthane,
- Ethylhexyl Methoxycinnamate,
- Isopropyl Méthoxy cinnamate,
- Isoamyl Méthoxy cinnamate
- Cinoxate,
- DEA Methoxycinnamate,
- Diisopropyle Methylcinnamate,
- Glyceryl Ethylhexanoate Dimethoxycinnamate
- Octocrylene,
- Etocrylene,
- Benzophenone-1,
- Benzophenone-2
- Benzophenone-3,
- Benzophenone-4,
- Benzophenone-5,
- Benzophenone-6,
- Benzophenone-8,
- Benzophenone-9,
- Benzophenone-12
- 3-Benzilidene camphor,
- 4-Methylbenzylidene camphor,
- Benzylidene Camphor Sulfonic Acid,
- Camphor Benzalkonium,
- Terephtalidene Dicamphor Sulfonic,
- Polyacrylamidomethyl Benzylidene Camphor,
- Phenylbenzimidazole Sulfonic Acid ,
- Benzimidazilate ,
- Anisotriazine,
- Ethylhexyl triazone,
- Diethylhexyl Butamido Triazone,
- Drometrizole Trisiloxane,
- Methylène bis-Benzotriazolyl Tetramethylbutylphénol,
- Menthyl anthranilate,
- Ethylhexyl Dimethoxybenzylidene Dioxoimidazoline Propionate,
- Polyorganosiloxane à fonctions benzalmalonate
et leurs mélanges.

44. Composition selon la revendications 43, **caractérisée par le fait que** le ou les filtres UV organiques sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
et leurs mélanges.

45. Composition selon l'une quelconque des revendications 1 à 44, où les filtres UV organiques sont présents dans les compositions allant de 0,2 à 15% en poids par rapport au poids total de la composition.

46. Composition selon l'une quelconque des revendications 1 à 45, **caractérisée par le fait qu'**elle comprend en outre, des pigments ou des nanopigments d'oxydes métalliques, enrobés ou non.

47. Composition selon la revendication 46, **caractérisée par le fait que** lesdits pigments ou nanopigments sont choisis parmi les oxydes de titane, de zinc, de fer, de zirconium, de cérium et leurs mélanges, enrobés ou non.

48. Composition selon l'une quelconque des revendications 1 à 47, **caractérisée par le fait qu'**elle comprend en outre au moins un agent de bronzage et/ou de brunissage artificiel de la peau.

49. Composition selon l'une quelconque des revendications 1 à 48, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères autres que ceux définis dans les revendications précédentes, les propulseurs, les agents alcalinisants ou acidifiants, les colorants.

50. Composition selon l'une quelconque des revendications 1 à 48, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

51. Composition selon l'une quelconque des revendications 1 à 48, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

52. Composition selon l'une quelconque des revendications 1 à 49, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

53. Utilisation de la composition définie selon l'une quelconque des revendications 1 à 52 pour la fabrication de compositions cosmétiques pour la protection de la peau et/ou des cheveux contre le rayonnement ultraviolet, en particulier le rayonnement solaire.

## Claims

1. Cosmetic composition, in particular for photoprotecting the skin and/or the hair, **characterized in that** it comprises, in a cosmetically acceptable support: (a) from 0.1% to 20% by weight, relative to the total weight of the composition, of at least one organic UV-screening agent and (b) at least one amphiphilic polymer comprising at least one ethylenically unsaturated monomer containing a sulfonic group, in free form or partially or totally neutralized form, and also at least one hydrophobic portion.

2. Composition according to Claim 1, **characterized in that** the hydrophobic portion of the amphiphilic polymer contains from 6 to 50 carbon atoms.

3. Composition according to Claim 2, **characterized in that** the hydrophobic portion of the amphiphilic polymer contains from 6 to 22 carbon atoms.

4. Composition according to Claim 3, **characterized in that** the hydrophobic portion of the amphiphilic polymer contains from 6 to 18 carbon atoms.

5. Composition according to Claim 4, **characterized in that** the hydrophobic portion of the amphiphilic polymer contains from 12 to 18 carbon atoms.

6. Composition according to any one of Claims 1 to 5, **characterized in that** the amphiphilic polymers are partially or totally neutralized with a mineral or organic base.

7. Composition according to any one of Claims 1 to 6, **characterized in that** the amphiphilic polymers have a number-average molecular weight ranging from 1000 to 20 000 000 g/mol.

8. Composition according to Claim 7, **characterized in that** the number-average molecular weight ranges from 20 000 to 5 000 000 g/mol.

9. Composition according to Claim 8, **characterized in that** the number-average molecular weight ranges from 100 000 to 1 500 000 g/mol.

10. Composition according to any one of the preceding claims, **characterized in that** the amphiphilic polymers are prepared by free-radical precipitation polymerization in tert-butanol.

11. Composition according to any one of the preceding claims, **characterized in that** the amphiphilic polymers are crosslinked or non-crosslinked.

12. Composition according to Claim 11, **characterized in that** the amphiphilic polymers are crosslinked.

13. Composition according to Claim 12, **characterized in that** the crosslinking agent(s) is(are) chosen from polyolefinically unsaturated compounds.

14. Composition according to Claim 13, **characterized in that** the crosslinking agent(s) is(are) chosen from methylenebisacrylamide, allyl methacrylate and trimethylolpropane triacrylate (TMPTA).

15. Composition according to any one of Claims 12 to 14, **characterized in that** the degree of crosslinking preferably ranges from 0.01 mol% to 10 mol% and more particularly from 0.2 mol% to 2 mol% relative to the polymer.

16. Composition according to any one of the preceding claims, **characterized in that** the ethylenically unsaturated monomer containing a sulfonic group is chosen from vinylsulfonic acid, styrenesulfonic acid, (meth)acrylamido(C₁-C₂₂)alkylsulfonic acids and N-(C₁-C₂₂)alkyl(meth)acrylamido-(C₁-C₂₂)alkylsulfonic acids, and the partially or totally neutralized forms thereof.

17. Composition according to Claim 16, **characterized in that** the ethylenically unsaturated monomer containing a sulfonic group is chosen from acrylamidomethanesulfonic acid, acrylamidoethanesulfonic acid, acrylamidopropanesulfonic acid, 2-acrylamido-2-methylpropanesulfonic acid, methacrylamido-2-methylpropanesulfonic acid, 2-acrylamido-n-butanesulfonic acid, 2-acrylamido-2,4,4-trimethylpentanesulfonic acid, 2-methacrylamidododecylsulfonic acid or 2-acrylamido-2,6-dimethyl-3-heptanesulfonic acid, and also the partially or totally neutralized forms thereof.

18. Composition according to either of Claims 16 and 17, **characterized in that** the ethylenically unsaturated monomer containing a sulfonic group is 2-acrylamido-2-methylpropanesulfonic acid (AMPS), and also the partially or totally neutralized forms thereof.

19. Composition according to Claim 18, **characterized in that** the amphiphilic polymers are chosen from random AMPS polymers modified by reaction with an n-mono (C₆-C₂₂) alkylamine or a di-n- (C₆-C₂₂)-alkylamine.

20. Composition according to either of Claims 18 and 19, **characterized in that** the amphiphilic AMPS polymers also contain at least one ethylenically unsaturated monomer not comprising a fatty chain.

21. Composition according to Claim 20, **characterized in that** the ethylenically unsaturated monomer not comprising a fatty chain is chosen from (meth)acrylic acids and the β-substituted alkyl derivatives thereof, and the esters thereof obtained with monoalcohols or mono- or polyalkylene glycols, or alternatively from (meth)acrylamides, vinylpyrrolidone, maleic anhydride, itaconic acid or maleic acid, or mixtures of these compounds.

22. Composition according to any one of Claims 1 to 18, **characterized in that** the amphiphilic AMPS polymers are chosen from amphiphilic copolymers of AMPS and of at least one ethylenically unsaturated hydrophobic monomer comprising at least one hydrophobic portion containing from 6 to 50 carbon atoms.

23. Composition according to Claim 22, **characterized in that** the hydrophobic portion contains from 6 to 22 carbon atoms.

24. Composition according to Claim 23, **characterized in that** the hydrophobic portion contains from 6 to 18 carbon atoms.

25. Composition according to Claim 24, **characterized in that** the hydrophobic portion contains from 12 to 18 carbon atoms.

26. Composition according to any one of Claims 22 to 25, **characterized in that** the ethylenically unsaturated hydrophobic monomer is chosen from the acrylates or the acrylamides of formula (I) below: 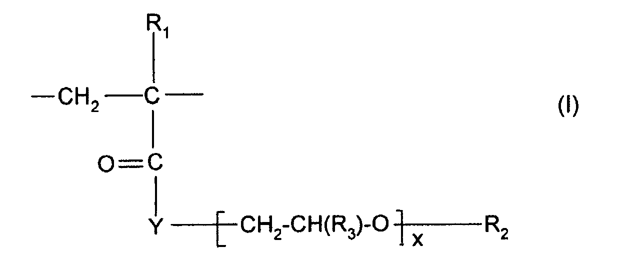 in which R₁ and R₃, which may be identical or different, denote a hydrogen atom or a linear or branched C₁-C₆ alkyl radical (preferably methyl); Y denotes O or NH; R₂ denotes a hydrophobic hydrocarbon-based radical containing at least from 6 to 50 carbon atoms, more preferably from 6 to 22 carbon atoms, even more preferably from 6 to 18 carbon atoms and more particularly from 12 to 18 carbon atoms; x denotes a number of moles of alkylene oxide and ranges from 0 to 100.

27. Composition according to Claim 26, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester; aromatic polycyclic groups.

28. Composition according to either of Claims 26 and 27, **characterized in that** the monomer of formula (I) also comprises at least one alkylene oxide unit (x ≥ 1).

29. Composition according to any one of Claims 26 to 28, **characterized in that** the monomer of formula (I) also comprises at least one polyoxyalkylenated chain.

30. Composition according to Claim 29, **characterized in that** the polyoxyalkylenated chain consists of ethylene oxide units and/or of propylene oxide units.

31. Composition according to Claim 30, **characterized in that** the polyoxyalkylenated chain consists solely of ethylene oxide units.

32. Composition according to any one of Claims 26 to 31, **characterized in that** the number of oxyalkylenated units ranges from 3 to 100.

33. Composition according to Claim 32, **characterized in that** the number of oxyalkylenated units ranges from 3 to 50.

34. Composition according to Claim 33, **characterized in that** the number of oxyalkylenated units ranges from 7 to 25.

35. Composition according to any one of Claims 22 to 27, **characterized in that** the amphiphilic AMPS polymer is chosen from:
- crosslinked or non-crosslinked, neutralized or non-neutralized copolymers comprising from 15% to 60% by weight of AMPS units and from 40% to 85% by weight of (C₈-C₁₆)alkyl(meth)acrylamide units or of (C₈-C₁₆)alkyl (meth)acrylate units, relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units, relative to the polymer.

36. Composition according to any one of Claims 22 to 27, **characterized in that** the amphiphilic AMPS polymer is chosen from:
- non-crosslinked copolymers of partially or totally neutralized AMPS and of n-dodecyl methacrylate,
- crosslinked or non-crosslinked copolymers of partially or totally neutralized AMPS and of n-dodecylmethacrylamide.

37. Composition according to Claims 22 to 34, **characterized in that** the amphiphilic AMPS polymer is chosen from copolymers consisting of 2-acrylamido-2-methylpropanesulfonic acid (AMPS) units of formula (II) below: 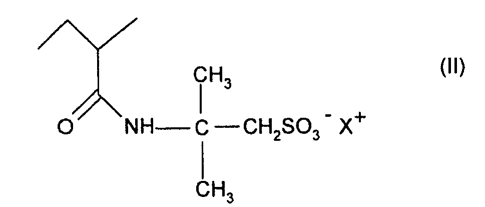 in which X⁺ is a proton, an alkali metal cation, an alkaline-earth metal cation or the ammonium ion, and of units of formula (III) below: 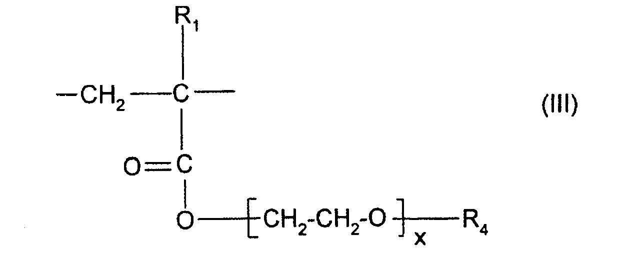 in which x denotes an integer ranging from 3 to 100, preferably from 5 to 80 and more preferably from 7 to 25; R₁ has the same meaning as that given above in formula (I) and R₄ denotes a linear or branched C₆-C₂₂ and more preferably C₁₀-C₂₂ alkyl.

38. Composition according to Claim 37, **characterized in that** x = 25, R₁ is methyl and R₄ is n-dodecyl.

39. Composition according to Claim 26 or 37, **characterized in that** the molar percentage proportion of units of formula (I) or of units of formula (III) in the polymers ranges from 50.1% to 99.9%.

40. Composition according to Claim 26 or 37, **characterized in that** the molar percentage proportion of units of formula (I) or of units of formula (III) in the polymers ranges from 0.1% to 50%.

41. Composition according to any one of the preceding claims, **characterized in that** the amphiphilic polymers are present in concentrations ranging from 0.01% to 30% by weight, more preferably from 0.1% to 10% by weight, even more preferably from 0.1% to 5% by weight and even more particularly from 0.5% to 2% by weight relative to the total weight of the composition.

42. Composition according to any one of Claims 1 to 41, **characterized in that** the organic UV-screening agent(s) is(are) chosen from anthranilates; cinnamic derivatives; dibenzoylmethane derivatives; salicylic derivatives; camphor derivatives; triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; dimers derived from α-alkylstyrene.

43. Composition according to Claim 42, **characterized in that** the organic UV-screening agent(s) is(are) chosen from the following compounds:
- PABA,
- Ethyl PABA,
- Ethyl Dihydroxypropyl PABA,
- Ethylhexyl Dimethyl PABA,
- Glyceryl PABA,
- PEG-25 PABA,
- Homosalate,
- Ethylhexyl Salicylate,
- Dipropyleneglycol Salicylate,
- TEA Salicylate,
- Butylmethoxydibenzoylmethane,
- Isopropyldibenzoylmethane,
- Ethylhexyl Methoxycinnamate,
- Isopropyl Methoxycinnamate,
- Isoamyl Methoxycinnamate,
- Cinoxate,
- DEA Methoxycinnamate,
- Diisopropyl Methylcinnamate,
- Glyceryl Ethylhexanoate Dimethoxycinnamate
- Octocrylene,
- Etocrylene,
- Benzophenone-1,
- Benzophenone-2,
- Benzophenone-3,
- Benzophenone-4,
- Benzophenone-5,
- Benzophenone-6,
- Benzophenone-8,
- Benzophenone-9,
- Benzophenone-12,
- 3-Benzylidenecamphor,
- 4-Methylbenzylidenecamphor,
- Benzylidenecamphorsulfonic Acid,
- Benzalkonium Camphor,
- Terephthalylidenedicamphorsulfonic acid,
- Polyacrylamidomethylbenzylidenecamphor,
- Phenylbenzimidazolesulfonic Acid,
- Benzimidazilate,
- Anisotriazine,
- Ethylhexyl Triazone,
- Diethylhexyl Butamido Triazone,
- Drometrizole Trisiloxane,
- Methylene bis-Benzotriazolyl Tetramethylbutylphenol,
- Menthyl anthranilate,
- Ethylhexyl Dimethoxybenzylidene Dioxoimidazoline Propionate,
- Polyorganosiloxane containing benzalmalonate functions,
and mixtures thereof.

44. Composition according to Claim 43, **characterized in that** the organic UV-screening agent(s) is(are) chosen from the following compounds:
- Ethylhexyl Salicylate,
- Butylmethoxydibenzoylmethane,
- Ethylhexyl Methoxycinnamate,
- Octocrylene,
- Phenylbenzimidazolesulfonic Acid,
- Terephthalylidenedicamphorsulfonic Acid,
- Benzophenone-3,
- Benzophenone-4,
- Benzophenone-5,
- 4-Methylbenzylidenecamphor,
- Benzimidazilate,
- Anisotriazine,
- Ethylhexyltriazone,
- Diethylhexylbutamidotriazone,
- Methylenebis(benzotriazolyl)tetramethylbutylphenol,
- Drometrizole trisiloxane,
and mixtures thereof.

45. Composition according to any one of Claims 1 to 44, in which the organic UV-screening agents are present in the compositions in proportions ranging from 0.2% to 15% by weight relative to the total weight of the composition.

46. Composition according to any one of Claims 1 to 45, **characterized in that** it also comprises coated or uncoated metal oxide pigments or nanopigments.

47. Composition according to Claim 46, **characterized in that** the said pigments or nanopigments are chosen from titanium oxide, zinc oxide, iron oxide, zirconium oxide and cerium oxide, and mixtures thereof, which may be coated or uncoated.

48. Composition according to any one of Claims 1 to 47, **characterized in that** it also comprises at least one agent for artificially tanning and/or browning the skin.

49. Composition according to any one of Claims 1 to 48, **characterized in that** it also comprises at least one adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellents, fragrances, preserving agents, surfactants, antiinflammatories, P substance antagonists, fillers, polymers other than those defined in the preceding claims, propellants, acidifying or basifying agents and colorants.

50. Composition according to any one of Claims 1 to 48, **characterized in that** it is a composition for protecting the human epidermis or an antisun composition and it is in the form of a nonionic vesicular dispersion, an emulsion, in particular an emulsion of oil-in-water type, a cream, a milk, a gel, a cream-gel, a suspension, a dispersion, a powder, a solid tube, a mousse or a spray.

51. Composition according to any one of Claims 1 to 48, **characterized in that** it is a makeup composition for the eyelashes, the eyebrows or the skin, and it is in solid or pasty, anhydrous or aqueous form or in the form of an emulsion, a suspension or a dispersion.

52. Composition according to any one of Claims 1 to 49, **characterized in that** it is a composition for protecting the hair against ultraviolet rays and it is in the form of a shampoo, a lotion, a gel, an emulsion or a nonionic vesicular dispersion.

53. Use of the composition defined according to any one of Claims 1 to 52 for the manufacture of cosmetic compositions for protecting the skin and/or the hair against ultraviolet radiation, in particular solar radiation.

## Patentansprüche

1. Kosmetische Zusammensetzung, insbesondere für den Lichtschutz der Haut und/oder der Haare, **dadurch gekennzeichnet, dass** sie in einem kosmetisch akzeptablen Träger enthält: (a) bezogen auf das Gesamtgewicht der Zusammensetzung 0,1 bis 20 Gew.-% mindestens eines organischen UV-Filters und (b) mindestens ein amphiphiles Polymer, das mindestens ein Monomer mit ethylenisch ungesättigter Bindung und einer in freier Form oder ganz oder teilweise neutralisiert vorliegenden Sulfonsäuregruppe enthält und ferner mindestens einen hydrophoben Bereich aufweist.

2. Zusammensetzung nach Anspruch 1, **dadurch gekennzeichnet, dass** der hydrophobe Bereich des amphiphilen Polymers 6 bis 50 Kohlenstoffatome enthält.

3. Zusammensetzung nach Anspruch 2, **dadurch gekennzeichnet, dass** der hydrophobe Teil des amphiphilen Polymers 6 bis 22 Kohlenstoffatome enthält.

4. Zusammensetzung nach Anspruch 3, **dadurch gekennzeichnet, dass** der hydrophobe Teil des amphiphilen Polymers 6 bis 18 Kohlenstoffatome enthält.

5. Zusammensetzung nach Anspruch 4, **dadurch gekennzeichnet, dass** der hydrophobe Teil des amphiphilen Polymers 12 bis 18 Kohlenstoffatome enthält.

6. Zusammensetzung nach einem der Ansprüche 1 bis 5, **dadurch gekennzeichnet, dass** die amphiphilen Polymere ganz oder teilweise mit einer anorganischen Base oder einer organischen Base neutralisiert sind.

7. Zusammensetzung nach einem der Ansprüche 1 bis 6, **dadurch gekennzeichnet, dass** die amphiphilen Polymere eine zahlenmittlere Molmasse von 1.000 bis 20.000.000 g/mol aufweisen.

8. Zusammensetzung nach Anspruch 7, **dadurch gekennzeichnet, dass** die zahlenmittlere Molmasse im Bereich von 20.000 bis 5.000.000 g/mol liegt.

9. Zusammensetzung nach Anspruch 8, **dadurch gekennzeichnet, dass** das die zahlenmittlere Molmasse im Bereich von 100 000 bis 1 500 000 g/mol liegt.

10. Zusammensetzung nach einem der vorhergehenden Ansprüche, **dadurch gekennzeichnet, dass** die amphiphilen Polymere durch radikalische Polymerisation unter Präzipitation in *tert*-Butanol hergestellt sind.

11. Zusammensetzung nach einem der vorhergehenden Ansprüche, **dadurch gekennzeichnet, dass** die amphiphilen Polymere vernetzt oder nicht vernetzt sind.

12. Zusammensetzung nach Anspruch 11, **dadurch gekennzeichnet, dass** die amphiphilen Polymere vernetzt sind.

13. Zusammensetzung nach Anspruch 12, **dadurch gekennzeichnet, dass** das Vernetzungsmittel oder die Vernetzungsmittel unter den Verbindungen mit mehreren olefinisch ungesättigten Bindungen ausgewählt sind.

14. Zusammensetzung nach Anspruch 13, **dadurch gekennzeichnet, dass** das Vernetzungsmittel oder die Vernetzungsmittel unter Methylen-bis-acrylamid, Allylmethacrylat oder Trimethylolpropantriacrylat (TMPTA) ausgewählt sind.

15. Zusammensetzung nach einem der Ansprüche 12 bis 14, **dadurch gekennzeichnet, dass** der Vernetzungsgrad vorzugsweise im Bereich von 0,01 bis 10 Mol-% und insbesondere im Bereich von 0,2 bis 2 Mol-%, bezogen auf das Polymer, liegt.

16. Zusammensetzung nach einem der vorhergehenden Ansprüche, **dadurch gekennzeichnet, dass** das Monomer mit ethylenisch ungesättigter Bindung und Sulfonsäuregruppe unter Vinylsulfonsäure, Styrolsulfonsäure, (Meth)acrylamido(C₁₋₂₂)alkylsulfonsäuren, N-(C₁₋₂₂)alkyl(meth)acrylamido(C₁₋₂₂)alkylsulfonsäuren sowie deren ganz oder teilweise neutralisierten Formen ausgewählt ist.

17. Zusammensetzung nach Anspruch 16, **dadurch gekennzeichnet, dass** das Monomer mit ethylenisch ungesättigter Bindung und Sulfonsäuregruppe unter Acrylamido-methan-sulfonsäure, Acrylamido-ethan-sulfonsäure, Acrylamido-propan-sulfonsäure, 2-Acrylamido-2-methylpropan-sulfonsäure, Methacrylamido-2-methylpropan-sulfonsäure, 2-Acrylamido-*n*-butan-sulfonsäure, 2-Acrylamido-2,4,4-trimethylpentan-sulfonsäure, 2-Methacrylamido-dodecyl-sulfonsäure, 2-Acrylamido-2,6-dimethyl-3-heptan-sulfonsäure sowie deren ganz oder teilweise neutralisierten Formen ausgewählt ist.

18. Zusammensetzung nach einem der Ansprüche 16 oder 17, **dadurch gekennzeichnet, dass** das Monomer mit ethylenisch ungesättigter Bindung und Sulfonsäuregruppe die 2-Acrylamido-2-methylpropan-sulfonsäure (AMPS) sowie ihre ganz oder teilweise neutralisierten Formen bedeutet.

19. Zusammensetzung nach Anspruch 18, **dadurch gekennzeichnet, dass** die amphiphilen Polymere unter den statistischen AMPS-Polymeren ausgewählt sind, die durch Umsetzung mit einem *n*-Mono(C₆₋₂₂)alkylamin oder Di-*n*-(C₆₋₂₂)alkylamin modifiziert wurden.

20. Zusammensetzung nach einem der Ansprüche 18 oder 19, **dadurch gekennzeichnet, dass** die amphiphilen AMPS-Polymere ferner mindestens ein Monomer mit ethylenisch ungesättigter Bindung enthalten, das keine Fettkette aufweist.

21. Zusammensetzung nach Anspruch 20, **dadurch gekennzeichnet, dass** das Monomer mit ethylenisch ungesättigter Bindung, das keine Fettkette aufweist, unter (Meth)acrylsäure und deren in β-Stellung Alkyl-substituierten Derivaten und ihren Estern, die mit Monoalkoholen oder Mono- oder Polyalkylenglycolen erhalten werden, oder (Meth)acrylamid, Vinylpyrrolidon, Maleinsäureanhydrid, Itaconsäure, Maleinsäure oder Gemischen dieser Verbindungen ausgewählt ist.

22. Zusammensetzung nach einem der Ansprüche 1 bis 18, **dadurch gekennzeichnet, dass** die amphiphilen Polymere unter den amphiphilen Copolymeren von AMPS und mindestens einem hydrophoben Monomer mit ethylenisch ungesättigter Bindung ausgewählt sind, das mindestens einen hydrophoben Bereich mit 6 bis 50 Kohlenstoffatomen aufweist.

23. Zusammensetzung nach Anspruch 22, **dadurch gekennzeichnet, dass** der hydrophobe Teil 6 bis 22 Kohlenstoffatome enthält.

24. Zusammensetzung nach Anspruch 23, **dadurch gekennzeichnet, dass** der hydrophobe Teil 6 bis 18 Kohlenstoffatome enthält.

25. Zusammensetzung nach Anspruch 24, **dadurch gekennzeichnet, dass** der hydrophobe Teil 12 bis 18 Kohlenstoffatome enthält.

26. Zusammensetzung nach einem der Ansprüche 22 bis 25, **dadurch gekennzeichnet, dass** das hydrophobe Monomer mit ethylenisch ungesättigter Bindung unter den Acrylaten oder Acrylamiden der folgenden Formel (I) ausgewählt ist: 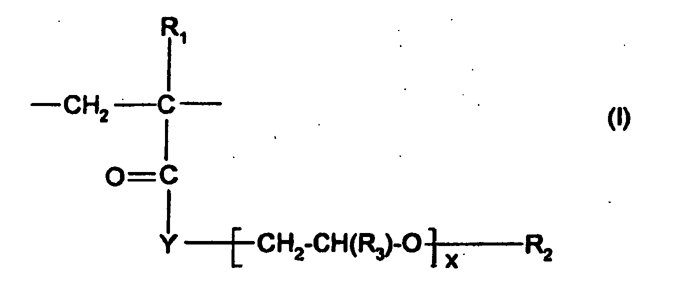 worin die Gruppen R₁ und R₃, die gleich oder verschieden sind, ein Wasserstoffatom oder eine geradkettige oder verzweigte C₁₋₆-Alkylgruppe bedeuten (vorzugsweise Methyl); Y O oder NH bedeutet; R₂ eine hydrophobe Kohlenwasserstoffgruppe mit zumindest 6 bis 50 Kohlenstoffatomen, vorzugsweise 6 bis 22 Kohlenstoffatomen, noch bevorzugter 6 bis 18 Kohlenstoffatomen und insbesondere 12 bis 18 Kohlenstoffatomen ist; x die Anzahl der Mol Alkylenoxid angibt und im Bereich von 0 bis 100 liegt.

27. Zusammensetzung nach Anspruch 26, **dadurch gekennzeichnet, dass** die hydrophobe Gruppe R₂ unter den geradkettigen, verzweigten oder cyclischen C₆₋₁₈-Alkylgruppen; C₆₋₁₈-perfluor-alkylierten Gruppen; der Cholesterylgruppe oder einem Cholesterinesterrest; und aromatischen polycyclischen Gruppen ausgewählt ist.

28. Zusammensetzung nach einem der Ansprüche 26 oder 27, **dadurch gekennzeichnet, dass** das Monomer der Formel (I) mindestens eine Alkylenoxideinheit aufweist (x ≥ 1).

29. Zusammensetzung nach einem der Ansprüche 26 bis 28, **dadurch gekennzeichnet, dass** das Monomer der Formel (I) ferner mindestens eine Polyoxyalkylenkette enthält.

30. Zusammensetzung nach Anspruch 29, **dadurch gekennzeichnet, dass** die Polyoxyalkylenkette aus Ethylenoxideinheiten und/oder Propylenoxideinheiten besteht.

31. Zusammensetzung nach Anspruch 30, **dadurch gekennzeichnet, dass** die Polyoxyalkylenkette ausschließlich aus Ethylenoxideinheiten besteht.

32. Zusammensetzung nach einem der Ansprüche 26 bis 31, **dadurch gekennzeichnet, dass** die Anzahl der Alkylenoxideinheiten im Bereich von 3 bis 100 liegt.

33. Zusammensetzung nach dem Anspruch 32, **dadurch gekennzeichnet, dass** die Anzahl der Alkylenoxidgruppen im Bereich von 3 bis 50 liegt.

34. Zusammensetzung nach dem Anspruch 33, **dadurch gekennzeichnet, dass** die Anzahl der Alkylenoxidgruppen im Bereich von 7 bis 25 liegt.

35. Zusammensetzung nach einem der Ansprüche 22 bis 27, **dadurch gekennzeichnet, dass** das amphiphile AMPS-Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamideinheiten oder (C₈₋₁₆)Alkyl(meth)acrylateinheiten, bezogen auf das Polymer, enthalten;
- Terpolymeren, die 10 bis 90 Mol-% Acrylamideinheiten, 0,1 bis 10 Mol-% AMPS-Einheiten und 5 bis 80 Mol-% n-(C₆₋₁₈)-Alkylacrylamideinheiten, bezogen auf das Polymer, enthalten.

36. Zusammensetzung nach einem der Ansprüche 22 bis 27, **dadurch gekennzeichnet, dass** das amphiphile AMPS-Polymer ausgewählt ist unter:
- nicht vernetzten Copolymeren von ganz oder teilweise neutralisierter AMPS und *n*-Dodecylmethacrylat;
- vernetzten oder nicht vernetzten Copolymeren von ganz oder neutralisierter AMPS und *n*-Dodecylmethacrylamid.

37. Zusammensetzung nach den Ansprüchen 22 bis 34, **dadurch gekennzeichnet, dass** das amphiphile AMPS-Polymer unter den Copolymeren ausgewählt ist, die aus 2-Acrylamido-2-methyl-propansulfonsäureeinheiten (AMPS) der folgenden Formel (II): 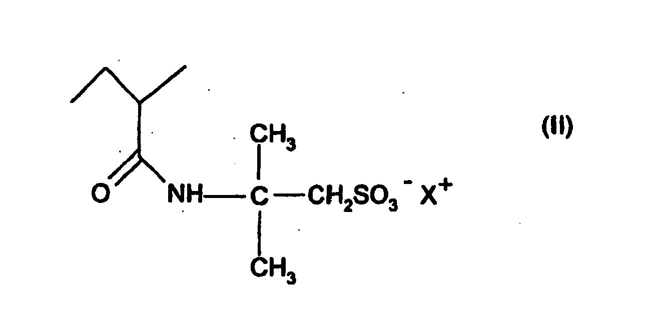 worin X⁺ ein Proton, ein Alkalimetallkation, ein Erdalkalimetallkation oder das Ammoniumion ist,
und Einheiten der folgenden Formel (III) bestehen: 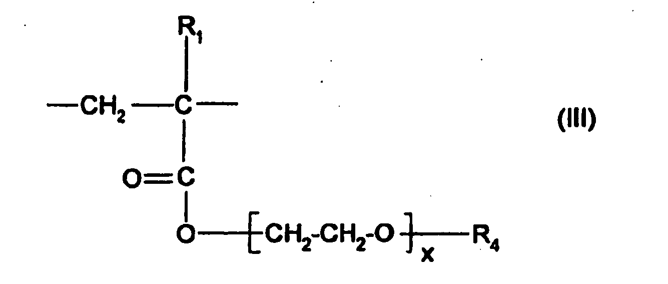 worin x eine ganze Zahl von 3 bis 100, vorzugsweise 5 bis 80 und noch bevorzugter 7 bis 25 bedeutet; R₁ die oben in Formel (I) angegebenen Bedeutungen hat und R₄ eine geradkettige oder verzweigte C₆₋₂₂-Alkylgruppe und vorzugsweise C₁₀₋₂₂-Alkylgruppe ist.

38. Zusammensetzung nach Anspruch 37, **dadurch gekennzeichnet, dass** x = 25, R₁ bedeutet Methyl und R₄ ist *n*-Dodecyl.

39. Zusammensetzung nach Anspruch 26 oder 37, **dadurch gekennzeichnet, dass** der prozentuale molare Anteil der Einheiten der Formel (I) oder der Einheiten der Formel (III) in den Polymeren im Bereich von 50,1 bis 99,9 % liegt.

40. Zusammensetzung nach Anspruch 26 oder 37, **dadurch gekennzeichnet, dass** der prozentuale molare Anteil der Einheiten der Formel (I) oder der Einheiten der Formel (III) in den Polymeren im Bereich von 0, 1 bis 50 % liegt.

41. Zusammensetzung nach einem der vorhergehenden Ansprüche, **dadurch gekennzeichnet, dass** die amphiphilen Polymere in Konzentrationen im Bereich von 0,01 bis 30 Gew.-%, vorzugsweise 0,1 bis 10 Gew.-%, noch bevorzugter 0,1 bis 5 Gew.-% und insbesondere 0,5 bis 2 Gew.-%, bezogen auf das Gesamtgewicht der Zusammensetzung, vorliegen.

42. Zusammensetzung nach einem der Ansprüche 1 bis 41, **dadurch gekennzeichnet, dass** der oder die organischen UV-Filter unter den Anthranilaten; Zimtsäurederivaten; Dibenzoylmethanderivaten; Salicylsäurederivaten; Campherderivaten; Triazinderivaten; Benzophenonderivaten; β,β'-Diphenylacrylatderivaten; Benzotriazolderivaten; Benzalmalonatderivaten; Benzimidazolderivaten; Imidazolinderivaten; Bis-benzoazolylderivaten; Derivaten von p-Aminobenzoesäure (PABA); Methylen-bis(hydroxyphenylbenzotriazol)derivaten; polymeren Filtern und Siliconfiltern; und von α-Alkylstyrol abgeleiteten Dimeren ausgewählt sind.

43. Zusammensetzung nach Anspruch 42, **dadurch gekennzeichnet, dass** der oder die organischen UV-Filter unter den folgenden Verbindungen ausgewählt sind:
- PABA
- Ethyl PABA
- Ethyl Dihydroxypropyl PABA
- Ethylhexyl Dimethyl PABA,
- Glyceryl PABA,
- PEG-25 PABA,
- Homosalate,
- Ethylhexyl Salicylate,
- Dipropyleneglycol Salicylate,
- TEA Salicylate,
- Butyl Methoxydibenzoylmethane,
- Isopropyl Dibenzoylmethane,
- Ethylhexyl Methoxycinnamate,
- Isopropyl Methoxycinnamate,
- Isoamyl Methoxycinnamate,
- Cinoxate,
- DEA Methoxycinnamate,
- Diisopropyl Methylcinnamate,
- Glyceryl Ethylhexanoate Dimethoxycinnamate,
- Octocrylene,
- Etocrylene,
- Benzophenone-1,
- Benzophenone-2,
- Benzophenone-3
- Benzophenone-4,
- Benzophenone-5,
- Benzophenone-6,
- Benzophenone-8,
- Benzophenone-9,
- Benzophenone-12,
- 3-Benzylidene Camphor,
- 4-Methylbenzylidene Camphor,
- Benzylidene Camphor Sulfonic Acid,
- Camphor Benzalkonium,
- Terephthalylidene Dicamphor Sulfonic,
- Polyacrylamidomethyl Benzylidene Camphor,
- Phenylbenzimidazole Sulfonic Acid,
- Benzimidazilate,
- Anisotriazine,
- Ethylhexyl Triazone,
- Diethylhexyl Butamido Triazone,
- Drometrizole Trisiloxane,
- Methylene bis-Benzotriazolyl Tetramethylbutylphenol,
- Menthyl Anthranilate,
- Ethylhexyl Dimethoxybenzylidene Dioxoimidazoline Propionate,
- Polyorganosiloxan mit Benzalmalonatfunktionen,
und deren Gemischen.

44. Zusammensetzung nach Anspruch 43, **dadurch gekennzeichnet, dass** der oder die organischen UV-Filter unter den folgenden Verbindungen ausgewählt sind:
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,
- Benzophenone-3,
- Benzophenone-4,
- Benzophenone-5,
- 4-Methylbenzylidene Camphor,
- Benzimidazilate,
- Anisotriazine,
- Ethylhexyl Triazone,
- Diethylhexyl Butamido Triazone,
- Methylene bis-Benzotriazolyl Tetramethylbutylphenol,
- Drometrizole Trisiloxane,
und deren Gemischen.

45. Zusammensetzung nach einem der Ansprüche 1 bis 44, **dadurch gekennzeichnet, dass** die organischen UV-Filter in den Zusammensetzungen 0,2 bis 15Gew.-%, bezogen auf das Gesamtgewicht der Zusammensetzung, ausmachen.

46. Zusammensetzung nach einem der Ansprüche 1 bis 45, **dadurch gekennzeichnet, dass** sie ferner Pigmente oder Nanopigmente von Metalloxiden enthält, die gegebenenfalls umhüllt sind.

47. Zusammensetzung nach Anspruch 46, **dadurch gekennzeichnet, dass** die Pigmente oder Nanopigmente unter den Oxiden von Titan, Zink, Eisen, Zirconium, Cer und deren Gemischen ausgewählt sind, wobei sie umhüllt oder nichtumhüllt sind.

48. Zusammensetzung nach einem der Ansprüche 1 bis 47, **dadurch gekennzeichnet, dass** sie ferner mindestens ein Bräunungsmittel und/oder Mittel zur künstlichen Braunfärbung der Haut enthält.

49. Zusammensetzung nach einem der Ansprüche 1 bis 48, **dadurch gekennzeichnet, dass** sie ferner mindestens einen Zusatzstoff enthält, der unter den Fettsubstanzen, organischen Lösungsmitteln, ionischen oder nichtionischen Verdickungsmitteln, beruhigenden Stoffen, Antioxidantien, Radikalfängern für freie Radikale, Trübungsmitteln, Stabilisatoren, Emollientien, Siliconen, α-Hydroxysäuren, Schaumverhütungsmitteln, Hydratisierungsmitteln, Vitaminen, Insekten abwehrenden Wirkstoffen, Parfums, Konservierungsmitteln, grenzflächenaktiven Stoffen, entzündungshemmenden Wirkstoffen, Substanz P-Antagonisten, Füllstoffen, Pigmenten, Polymeren, die von den in den vorherigen Ansprüchen definierten Polymeren verschieden sind, Treibmitteln, Alkalisierungsmitteln oder Ansäuerungsmitteln und Farbmittel ausgewählt ist.

50. Zusammensetzung nach einem der Ansprüche 1 bis 48, **dadurch gekennzeichnet, dass** es sich um eine Zusammensetzung zum Schutz der menschlichen Epidermis oder ein Sonnenschutzmittel handelt und sie in Form einer nichtionischen Vesikeldispersion, als Emulsion und insbesondere Emulsion vom Öl-in-Wasser-Typ, Creme, Milch, Gel, Gelcreme, Suspension, Dispersion, Pulver, fester Stift, Schaum oder Spray vorliegt.

51. Zusammensetzung nach einem der Ansprüche 1 bis 48, **dadurch gekennzeichnet, dass** es sich um eine Zusammensetzung zum Schminken der Wimpern, der Augenbrauen oder der Haut handelt und sie in fester oder pastöser, wässriger oder wasserfreier Form, als Emulsion, Suspension oder Dispersion vorliegt.

52. Zusammensetzung nach einem der Ansprüche 1 bis 49, **dadurch gekennzeichnet, dass** es sich um eine Zusammensetzung handelt, die zum Schutz der Haare gegen UV-Strahlung vorgesehen ist, und dadurch, dass sie in Form eines Haarwaschmittels, einer Lotion, eines Gels, einer Emulsion oder einer nichtionischen Vesikeldispersion vorliegt.

53. Verwendung der in einem der Ansprüche 1 bis 52 definierten Zusammensetzung für die Herstellung von kosmetischen Zusammensetzungen zum Schutz der Haut und/oder der Haare gegen UV-Strahlung und insbesondere Sonnenlicht.
